# EUROPEAN PATENT APPLICATION

(11) **EP 2 444 913 A1**
(43) Date of publication of application: **25.04.2012**
(21) Application number: 11186135.7
(22) Date of filing: 21.10.2011
(51) Int. Cl.: G06F 19/26

(54) **Method for providing genetic information and genetic information server using the same**

(30) Priority: 21.10.2010 KR 20100102779
(71) Applicant: Samsung SDS Co., Ltd., Seoul 135-082 (KR)
(72) Inventor: Park, Minseo, 135-248 Seoul (KR); Kim, Woo-Yeon, 138-864 Seoul (KR); Choi, Jeong A., 135-798 Seoul (KR); Kwon, Moon Young, 135-798 Seoul (KR); Kim, Bomi, Gyeonggi-do (KR)
(74) Representative: Grünecker, Kinkeldey, Stockmair & Schwanhäusser

(57) **Abstract**

A method for providing genetic information, a genetic information server, and a non-transitory computer readable medium in which a genetic information browser program is recorded are provided. The method includes: displaying a plurality of tracks of attribute information relating to a plurality of tracks of genetic information on a plurality of default tracks, respectively, generating a private track, selecting at least two tracks from the plurality of tracks of attribute information displayed on the default tracks; and combining the selected at least two tracks of attribute information and displaying the combined attribute information on the generated private track Accordingly, an intuitive genetic information browser interface is provided.

## Description

### CROSS-REFERENCE TO RELATED PATENT APPLICATION

This application claims priority from Korean Patent Application No. 10-2010-0102779, filed on October 21, 2010, in the Korean Intellectual Property Office, the disclosure of which is hereby incorporated by reference in its entirety.

### BACKGROUND

### 1. Field

Apparatuses and methods consistent with exemplary embodiments relate to a method for providing genetic information and a genetic information server using the same, and more particularly, to a method for providing genetic information which can improve user convenience, and a genetic information server using the same.

### 2. Description of the Related Art

A correlation between genetic information and diseases has been discovered and the importance of a study on a genomic sequence is increasingly stressed. Many researchers or users are increasingly demanding genomic sequence and variation information.

However, since an amount of data relating to the genomic sequence and the variation information is huge, the researchers or users encounter difficulties in using desired information. For this reason, various methods for more efficiently providing data relating to a genomic sequence and variation information have been suggested.

For example, various genome browsers to display and explore genomic data such as a UCSC genome browser, an NCBI map viewer, an ensemble genome browser, a generic genome browser and a RIKEN OMICS browser are provided.

However, the related-art browsers are not able to display results of various samples on a single track simultaneously and have difficulty in displaying the results interactively in a format that a researcher or a user desires and that is accessible in real time.

### SUMMARY

One or more aspects of the exemplary embodiments provide a method in which a user can customize genetic information interactively by using a method of combining and displaying various genetic information, a genetic information server, and/or a medium (such as a computer, mobile, and etc.) which can display or write a genetic information browser program.

According to an aspect of an exemplary embodiment, there is a method for providing genetic information, the method comprising: displaying a plurality of tracks of attribute information relating to a plurality of tracks of genetic information on a plurality of default tracks, respectively; generating a private track; selecting at least two tracks from the plurality of tracks of attribute information displayed on the default tracks; and combining the selected at least two tracks of attribute information and displaying the combined attribute information on the generated private track.

According to an aspect of an exemplary embodiment, there is a method for providing genetic information, the method including: displaying one or more tracks which display different genetic information; generating one or more "customer tracks" which can be interactively customized by a user, selecting two or more tracks that can display different attributes (two genetic information); and combining selected tracks (genetic information) into a customer track.

According to an aspect of an exemplary embodiment, there is a genetic information server comprising: a communication unit which transmits a plurality of tracks of attribute information relating to a plurality of tracks of genetic information to be displayed on a plurality of default tracks to a terminal apparatus; a combination unit which, if a request for combination of at least two tracks of attribute information among the transmitted plurality of tracks of attribute information is received from the terminal apparatus, combines the requested at least two tracks of attribute information; and a controller which transmits the combined attribute information to the terminal apparatus through the communication unit.

According to an aspect of another exemplary embodiment, there is provided a non-transitory computer readable medium on which a genetic information browser program is recorded to perform operations of: displaying a plurality of tracks of attribute information relating to a plurality of genetic information on a plurality of default tracks, respectively, generating a private track (customer track), selecting at least two tracks of the plurality of tracks of attribute information displayed on the default tracks; and combining the selected at least two tracks of attribute information and displaying the combined attribute information on the generated private track.

According to an aspect of another exemplary embodiment, there is provided a method for providing genetic information, the method comprising: generating a private track on which a user can define and customize genetic information; and displaying genetic information defined and customized by a user on the generated private track.

Exemplary embodiments may include any, all or none of the following advantages:

According to the method for providing genetic information, the genetic information server using the same, or the non-transitory computer readable medium in which the genetic information browser program is recorded, the user can easily select desired genetic information and can combine attribute information relating to the selected genetic information as he/she desires. Accordingly, the user can intuitively compare and analyze a genomic sequence, related information, and variation information. A convenient and intuitive interface is provided so that even a user unfamiliar to a computer can easily use it.

Additional aspects and advantages of the exemplary embodiments will be set forth in the detailed description, will be obvious from the detailed description, or may be learned by practicing the exemplary embodiments.

### BRIEF DESCRIPTION OF THE DRAWINGS

The above and other features and advantages will become more apparent by describing in detail exemplary embodiments with reference to the attached drawings in which:

FIG. 1 is a network configuration view of a genetic information providing system according to an exemplary embodiment;

FIG. 2 is a block diagram illustrating a genetic information server according to an exemplary embodiment;

FIG. 3 is a view illustrating a user interface (Ul) to provide genetic information according to an exemplary embodiment;

FIG. 4 is a view illustrating an example of the Ul of FIG. 3 when it is used;

FIG. 5 is a flowchart illustrating a method for providing genetic information according to an exemplary embodiment; and

FIG. 6 is a flowchart illustrating a method for providing genetic information according to another exemplary embodiment.

### DETAILED DESCRIPTION OF EXEMPLARY EMBODIMENTS

Exemplary embodiments will now be described more fully with reference to the accompanying drawings to clarify aspects, features and advantages of the inventive concept. The exemplary embodiments may, however, be embodied in many different forms and should not be construed as limited to the exemplary embodiments set forth herein. Rather, the exemplary embodiments are provided so that this disclosure will be thorough and complete, and will fully convey the scope of the application to those of ordinary skill in the art. It will be understood that when an element, layer or region is referred to as being "on" another element, layer or region, the element, layer or region can be directly on another element, layer or region or intervening elements, layers or regions.

Also, it will be understood that when a first element (or first component) is referred to as being operated or executed "on" a second element (or second component), the first element (or first component) can be operated or executed in an environment where the second element (or second component) is operated or executed or can be operated or executed by interacting with the second element (or second component) directly or indirectly.

Also, it will be understood that when an element, component, apparatus or system is referred to as comprising a component consisting of a program or software, the element, component, apparatus or system can comprise hardware (for example, a memory or a central processing unit (CPU)) necessary for executing or operating the program or software or another program or software (for example, a operating system (OS), a driver necessary for driving hardware), unless the context clearly indicates otherwise.

Also, it will be understood that an element (or component) can be realized by software, hardware, or software and hardware, unless the context clearly indicates otherwise.

The terms used herein are for the purpose of describing particular exemplary embodiments only and are not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, layers, regions, elements, components, and/or groups thereof, but do not preclude the presence or addition of one or more other features, integers, steps, operations, layers, regions, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

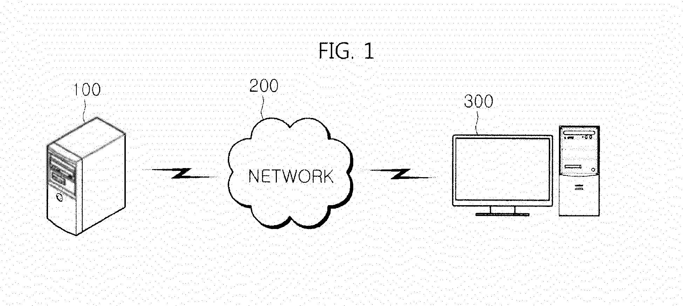

FIG. 1 is a network configuration view illustrating a genetic information providing system according to an exemplary embodiment.

Referring to FIG. 1, a genetic information providing system according to an exemplary embodiment may include a genetic information server 100 and at least one terminal apparatus 300 which are connected to each other through a network 200. The network 200 may a wired and/or a wireless network. According to the exemplary embodiment, a user can customize genetic information interactively.

The genetic information server 100 is a server that provides genetic information to the terminal apparatus 300 which accesses the genetic information server 100 through the network 200. To achieve this, the genetic information server 100 creates a database with a plurality of tracks of genetic information and manages the database. The term "manage" recited herein may be interpreted as meaning storing, correcting, deleting and/or adding data for the purpose of explaining the present inventive concept.

If the genetic information server 100 receives a request for at least one piece of genetic information from the terminal apparatus 300, the genetic information server 100 may provide attribute information relating to requested genetic information to the terminal apparatus 300. At this time, the genetic information server 100 may transmit the attribute information to the terminal apparatus 300 in a web page format that can be read by a web browser or in a format that can be read by a dedicated application. The dedicated application according to an exemplary embodiment refers to a dedicated genetic information browser that can provide genetic information. If the genetic information server 100 has a web server function of providing the attribute information relating to the genetic information in the web page format, the dedicated application is a genetic information browser having a web browser function. On the other hand, if the genetic information server 100 does not have the web server function, the dedicated application is a dedicated browser that displays genetic information stored in the genetic information server 100 on the terminal apparatus 300.

The genetic information recited herein may be a DNA (Deoxyribonucleic acid) sequence and the attribute information may be, for example, genes and variations such as SNP (Single Nucleotide Polymorphism) or repeat elements.

According to an exemplary embodiment, if the genetic information server 100 has the web server function, the genetic information server 100 may transmit to the terminal apparatus 300 the attribute information of the format that can be read by a web browser installed in the terminal apparatus 300 or a dedicated application in which a web browser is embedded.

After transmitting the attribute information to the terminal apparatus 300, the genetic information server 100 may receive a request for a combination of at least two tracks of attribute information and an area to be combined from the terminal apparatus 300. In this case, the genetic information server 100 combines the at least two tracks of attribute information with respect to the area to be combined and transmits the combined attribute information to the terminal apparatus 300.

According to another exemplary embodiment, the genetic information server 100 may provide the genetic information to the terminal apparatus 300 in a flash format.

According to an exemplary embodiment, the genetic information server 100 and the terminal apparatus 300 may communicate with each other through a web browser or a dedicated application. The web browser recited herein may be a general browser that is already installed in the terminal apparatus 300, such as NETSCAPE, MICROSOFT INTERNET EXPLORER, MOZILLA FIREFOX, or GOOGLE CHROME, and the dedicated application may be an application that is developed to dedicatedly display genetic information.

When the terminal apparatus 300 exchanges information with the genetic information server 100 using the web browser, a separate dedicated application need not be installed. On the other hand, if the terminal apparatus 300 exchanges information with the genetic information server 100 using the dedicated application, a process of installing the dedicated application may be needed at least one time. According to an exemplary embodiment, the terminal apparatus 300 may receive a setup file of the dedicated application from the genetic information server 100 and install the dedicated application or may receive the setup file of the dedicated application from a user directly and install the dedicated application.

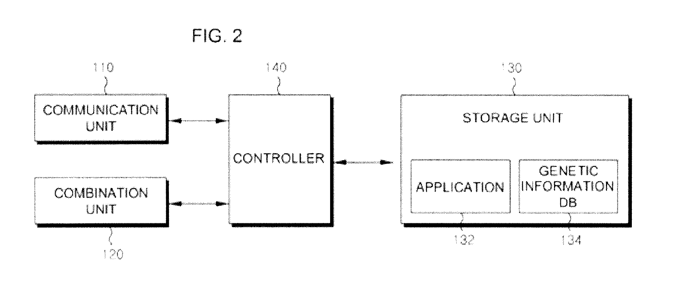

The genetic information server 100 will be explained in detail below with reference to FIG. 2.

The terminal apparatus 300 is an apparatus that is used by a user who wants to receive genetic information, and accesses the genetic information server 100 through the network 200 and obtains the genetic information from the genetic information server 100.

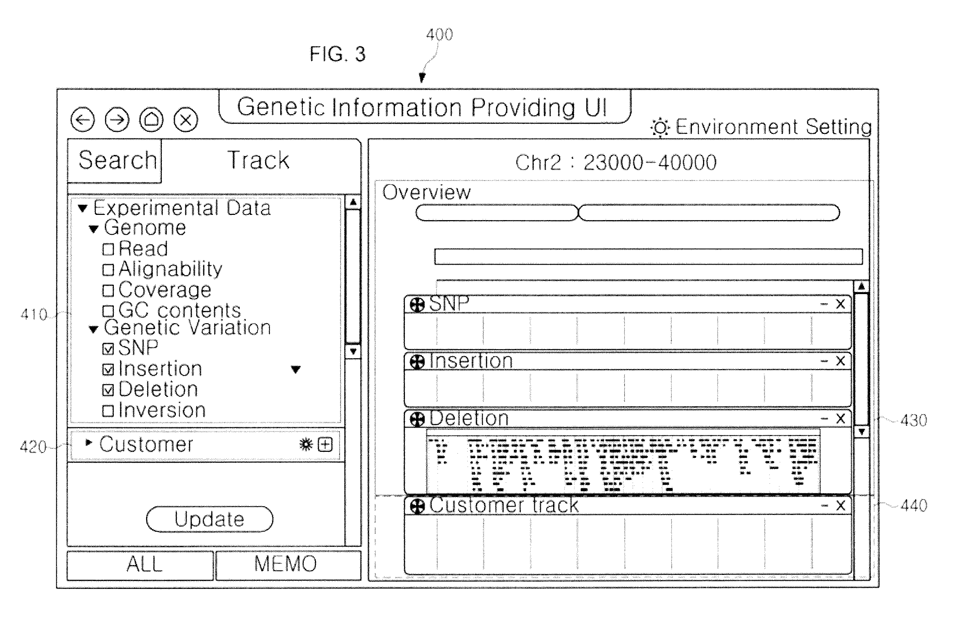

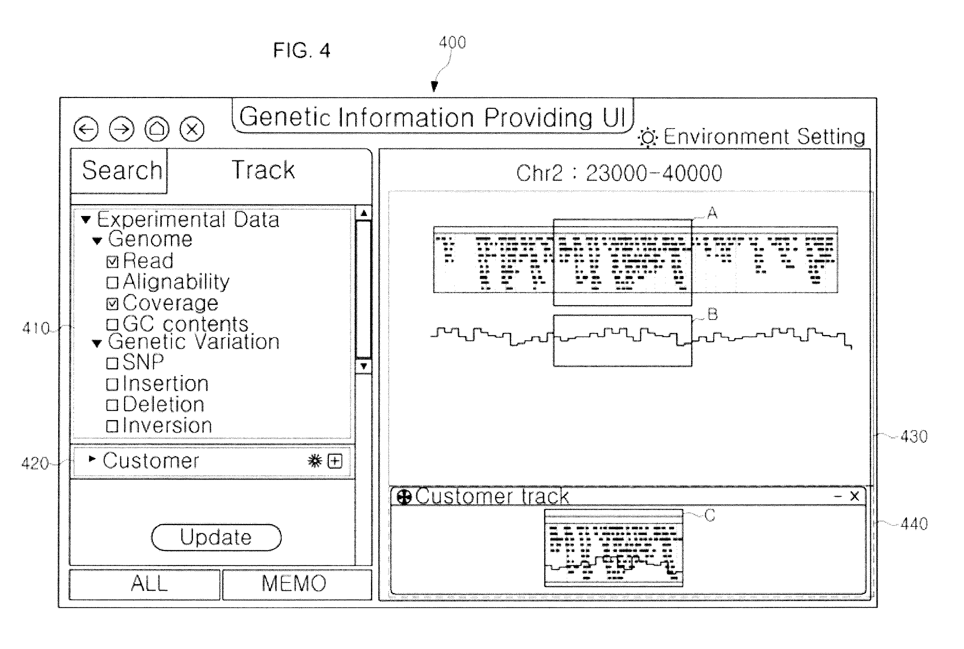

The terminal apparatus 300 may be a personal computer, a notebook computer, a smart phone, iPad, or S-pad. Any type of apparatus that is capable of operating a web browser or a dedicated application may be used as the terminal apparatus 300. The web browser or the dedicated application will be explained in detail below with reference to FIGS. 3 and 4.

According to an exemplary embodiment, although not shown, the terminal apparatus 300 may include a central processing unit (CPU), a user input/output device (for example, a monitor, a keyboard, or a mouse), and a storage unit. The web browser or the dedicated application may be stored in the storage unit and executed under control of the CPU, so that genetic information providing user interface (Ul) can be displayed on the user output device such as a monitor.

The terminal apparatus 300 may include an input device such as a keyboard or a mouse and an output device such as a monitor. The terminal apparatus 300 may include a device combining input and output devices such as a touch screen. The monitor may display a Ul provided by the web browser or the dedicated application, receive a predetermined manipulation signal from the user through a device such as a keyboard, a mouse, or a touch screen, and receive genetic information to be requested from the genetic information server 100 through the web browser or the dedicated application. After that, the information provided from the genetic information server 100 may be displayed on the monitor by the web browser or the dedicated application.

FIG. 2 is a block diagram illustrating the genetic information server according to an exemplary embodiment.

Referring to FIG. 2, the genetic information server 100 according to an exemplary embodiment may include a communication unit 110, a combination unit 120, a storage unit 130, and a controller 140.

The communication unit 110 supports an interface with the network 200 and the genetic information server 100 communicates with the terminal apparatus 300 through the communication unit 110. However, the communication unit 110 may be operated under control of the controller 140.

In this embodiment, the communication unit 110 may receive an access request and a genetic information request from the terminal apparatus 300 and may transmit the genetic information and the related attribute information to the terminal apparatus 300 in response to the requests. The communication unit 110 may receive a request for a combination of a plurality of tracks of attribute information and an area to be combined from the terminal apparatus 300, and may transmit combined attribute information to the terminal apparatus 300 in response to this request. The area to be combined refers to an area that is to be combined when two or more tracks of attribute information are combined.

If the request for a combination of the plurality of tracks of attribute information, that is, at least two tracks of attribute information, is received from the terminal apparatus 300, the combination unit 120 combines attribute information corresponding to the area requested to be combined. When combining, the combination unit 120 overlaps the attribute information corresponding to the area to be combined, while displaying all of the plurality of overlapping tracks of attribute information.

For example, it is assumed that a base sequence is 1000bp long and consists of base numbers 1, 2, 3, ...., 999, 1000. It is also assumed that first attribute information (SNP) pertains to a base sequence consisting of base numbers 1 to 200, and second attribute information (repeat element) pertains to a base sequence consisting of base numbers 150 to 400.

In this case, if the first attribute information and the second attribute information are combined with each other according to an exemplary embodiment, a portion where the first attribute information and the second attribute information overlap each other may be displayed. In other words, with respect to the base sequence consisting of base numbers 150 to 200, the SNP and the repeat element information may be displayed on a user track.

Alternatively, if the first attribute information and the second attribute information are combined, not only the portion where the first attribute information and the second attribute information overlap each other, but also a portion where the first attribute information and the second attribute information does not overlap each other may be displayed. In other words, the base sequence consisting of base numbers 1 to 400 is displayed on the user track and the SNP and the repeat element of base numbers 150 to 200 may be displayed on the user track.

For another example, it is assumed that the first attribute information (SNP) pertains to a base sequence consisting of base numbers 1 to 200 and the second attribute information (repeat element) also pertains to the base sequence consisting of base numbers 1 to 200. In this case, the base sequence consisting of base numbers 1 to 200 is displayed on the user track and the SNP and the repeat element of base numbers 1 to 200 are displayed on the user track.

The storage unit 130 may store information necessary for driving the genetic information server 100. For example, the storage unit 130 may include at least one application 132 and genetic information database 134. The application 132 may be a dedicated application that receives genetic information and attribute information of the genetic information from the genetic information server 100 and displays the information, and the genetic information may be realized by a database such as the genetic information DB 134.

According to an exemplary embodiment, the application stored in the storage unit 130 may be transmitted to the terminal apparatus 300 and may be installed in the terminal apparatus 300.

The genetic information DB 134 may store a plurality of tracks of genetic information and attribute information relating to each of the plurality of tracks of genetic information. The attribute information relating to the genetic information may be data that has a predetermined pattern according to a characteristic of each gene.

In this specification, the storage unit 130 means all storing means for storing data owned by the genetic information server 100. For example, the genetic information server 100 may include a plurality of storing means for storing data temporarily and permanently such as a flash memory, a RAM, a ROM, a hard disk, a magnetic tape, a floppy disk, an optical data storage device, or a register, and the plurality of storing means are collectively called the storage unit 130.

The controller 140 controls an overall operation of the genetic information server 100. That is, the controller 140 may control an interactive operation among the communication unit 110, the combination unit 120, and the storage unit 130.

The controller 140 may control the communication unit 110 to transmit specific information to the terminal apparatus 300. Also, if a request for combination and an area to be combined are received from the terminal apparatus 300 through the communication unit 110, the controller 140 may control the combination unit 120 to combine the area.

Also, if there is information to be transmitted to the terminal apparatus 300 or if a request for transmission of specific information is received from the terminal apparatus 300, the controller 140 may extract data to be transmitted from the storage unit 130.

FIG. 3 is a view illustrating a Ul to provide genetic information according to an exemplary embodiment.

Referring to FIG. 3, a basic format of a genetic information providing Ul 400 will be explained. In this embodiment, the term 'genetic information providing Ul' 400 is used for convenience of explanation but it should be understood that this term does not limit the concept of the inventive concept.

The genetic information providing Ul 400 shown in FIG. 3 is an example of genetic information providing Ul displayed for a user by a web browser or a dedicated application included in the terminal apparatus 300. For example, if the terminal apparatus 300 includes a web browser, the web browser receives a web page capable of displaying the genetic information providing Ul 400 from the genetic information server 100 and displays the web page for the user. Alternatively, if the terminal apparatus 300 includes a dedicated application, the application receives data (or information) necessary for displaying the genetic information providing Ul 400 from the genetic information server 100 and displays the data for the user. According to this embodiment, a user can define and customize genetic information in (or on) the genetic information providing Ul.

The genetic information providing Ul 400 includes a list of display area 410 to display a list of a plurality of tracks of genetic information in such a format that some of the tracks of information is selected, a default track area 430 to display a plurality of default tracks, and a private track area 440 (marked "customer track" in FIG. 3) to display a private track in (or on) which a user can define and customize genetic information.

In this specification, the term 'customer track' equivalent to the term 'private track' and may be used interchangeably. The default track area is distinguished from the private track area and displays genetic information that is always selected unless the user gives a specific command. As shown in FIG. 3, the list display area 410 is formed on one side of the genetic information providing Ul 400 in a vertical direction, and the default track area 430 and the private track area 440 are formed on the other side of the genetic information providing Ul 400 in a horizontal direction.

The private track area 440 may not be displayed on the genetic information providing Ul 400 of the basic format and may be an empty space. This is because the private track of the private track area 440 is generated at the user's request. Alternatively, the private track area 440 may be displayed indistinguishably from the default track area 430, and, if the user wishes to generate a private track afterward, some of the default track area 430 may be changed to the private track area 440.

Also, the genetic information providing Ul 400 may further include a private track generation requesting menu 420 provided on one side thereof to request generation of a private track. The number of private tracks to be generated is determined according to the number of clicks on a "+" button of the private track generation requesting menu 420 through the genetic information providing Ul 400.

The genetic information providing Ul 400 shown in FIG. 3 includes a 'Search' tab and a 'Track' tab. The 'Track' tab supports the list display area 410 to display the list of the plurality of tracks of genetic information in such a format that some of the tracks of information are selected. Although not shown, the 'Search' tab may include a menu through which the user can search desired genetic information by inputting a name of the genetic information directly.

FIG. 4 is a view illustrating an example of the genetic information providing Ul of FIG. 3 when it is used.

The user executes the web browser or the dedicated application by manipulating the terminal apparatus 300. Accordingly, the monitor of the terminal apparatus 300 displays the genetic information providing Ul 400 of the basic format shown in FIG. 3. The user may select a plurality of names of genetic information (at least two names) from the list display area 410 to display attribute information of the genetic information using a mouse which is a general input device. For example, 'Read' and 'Coverage' may be selected.

If the user selects the plurality of tracks of genetic information, the web browser or the dedicated application requests attribute information relating to the genetic information selected by the user from the genetic information server 100 and receives the attribute information. After that, the web browser or the dedicated application displays the attribute information relating to the genetic information received from the genetic information server 100 on the default track area 430 of the genetic information providing Ul 400.

As shown in FIG. 4, the two tracks of genetic information are selected from the list display area 410 and accordingly the attribute information relating to the selected genetic information is displayed on each of the two default tracks on the default track area 430.

After that, if the user wants to combine at least some of the attribute information relating to the two tracks of genetic information displayed on the default tracks, the user selects a desired area from each default track and sets an area to be combined using a mouse. As shown in FIG. 4, an area A is set to be combined in the first attribute information, and an area B is set to be combined in the second attribute information. After that, if an 'ALL' button is clicked, the web browser or the dedicated application requests the genetic information server 100 to combine the attribute information. Accordingly, the web browser or the dedicated application overlaps the attribute information on the area A and the area B which are combined with each other by the genetic information server 100 and displays the combined attribute information C on the same position of the private track.

If the user presses the 'ALL' button without setting the area to be combined, the web browser or the dedicated application may set all tracks of attribute information of genetic information displayed on the default tracks as the area to be combined.

In the above embodiment, the user is provided with the combined attribute information through the operations of selecting some tracks of attribute information from the attribute information displayed on the default tracks through the web browser or the dedicated application and then clicking the 'ALL' button.

In a modified example, a combination of two tracks of attribute information may be requested by dragging the area A which has been set to be combined using the mouse and dropping it to the private track and dragging the area B set to be combined and dropping it to the private track.

As shown in FIG. 4, some of the tracks, that is, some of the plurality of default tracks in the default track area 430 and some of the plurality of private tracks in the private track area 440 may be deleted according to a user's selection. Also, some of the tracks may be minimized according to a user's selection so that they disappear from only the current screen viewed by the user. To achieve this, each track is provided with a button to minimize and delete the track. Also, the user may move each track to a predetermined position on the current screen by dragging it using the mouse.

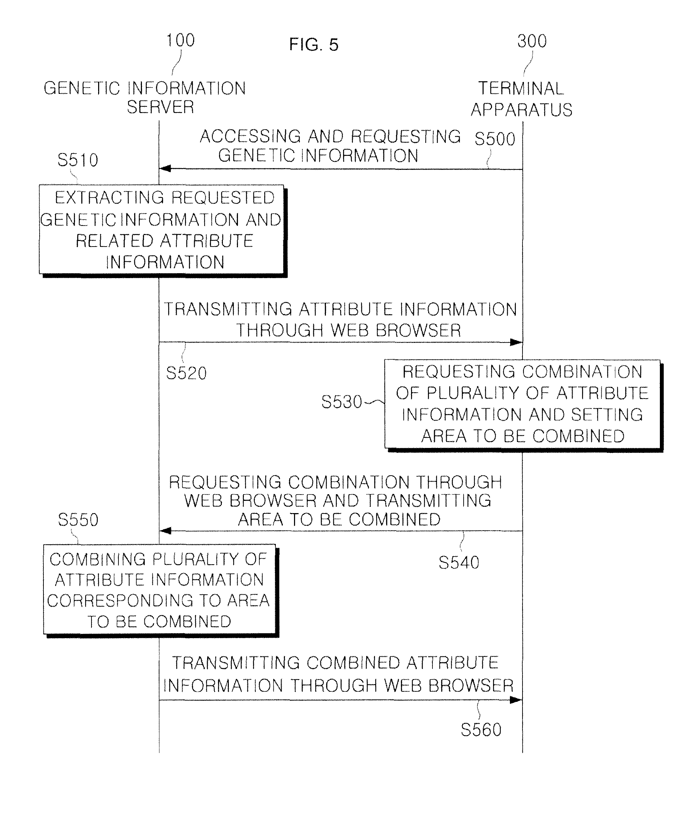

FIG. 5 is a flowchart illustrating a method for providing genetic information according to an exemplary embodiment.

Referring to FIGS. 1 through 5, a method for providing genetic information according to an exemplary embodiment will be explained. According to the exemplary embodiment, a user can customize genetic information interactively. In this embodiment, the terminal apparatus 300 accesses the genetic information server 100 through a web browser.

The terminal apparatus 300 executes the web browser and then access the genetic information server 100 through the web browser. If the terminal apparatus 300 accesses the genetic information server 100, the genetic information server 100 provides the genetic information providing Ul 400 to the web browser of the terminal apparatus 300, and the terminal apparatus 300 receives selection of genetic information on the list display area 410 from the user and requests the genetic information server 100 to provide the selected genetic information (S500).

The genetic information server 100 extracts attribute information relating to the requested genetic information from the genetic information DB 134 to provide the genetic information requested by the terminal apparatus 300 (S510).

The genetic information server 100 transmits the genetic information providing Ul 400 in which the attribute information extracted from the genetic information DB 134 is inserted into each of the default tracks to the terminal apparatus 300 (S520).

The terminal apparatus 300 receives the genetic information providing Ul in which the attribute information is inserted into each of the default tracks from the genetic information server 100 and displays the genetic information providing Ul on the monitor. By doing so, the user may request a combination of some of the attribute information displayed on the default tracks and may set an area to be combined. After that, if the user sets the genetic attribute information to be combined and the area to be combined through the terminal apparatus 300 (S530), the terminal apparatus 300 requests combination by transmitting the genetic attribute information and the area to be combined to the genetic information server 100 (S540).

The genetic information server 100 combines the plurality of tracks of attribute information corresponding to the area requested to be combined by the terminal apparatus 300 (S550), and transmits the genetic information providing Ul in which a result of combination is inserted into the private track to the terminal apparatus 300 (S560).

Through the above process, the user can easily obtain the attribute information relating to the genetic information from the genetic information server 100 through the general web browser, and can combine at least two tracks of attribute information as he/she wants and can identify the combined attribute information on a single track.

In the embodiment of FIG. 5, the genetic information server 100 transmits the genetic information providing Ul 400 in which the attribute information extracted from the genetic information DB 134 is inserted into each of the default tracks to the terminal apparatus 300. However, alternatively, the genetic information server 100 may transmit the attribute information extracted from the genetic information DB 134 to the terminal apparatus 300 and the terminal apparatus 300 may insert the attribute information received from the genetic information server 100 to each of the default tracks and may display the attribute information.

According to the embodiment of FIG. 5, a user can define and customize genetic information in (or on) the genetic information providing Ul 400.

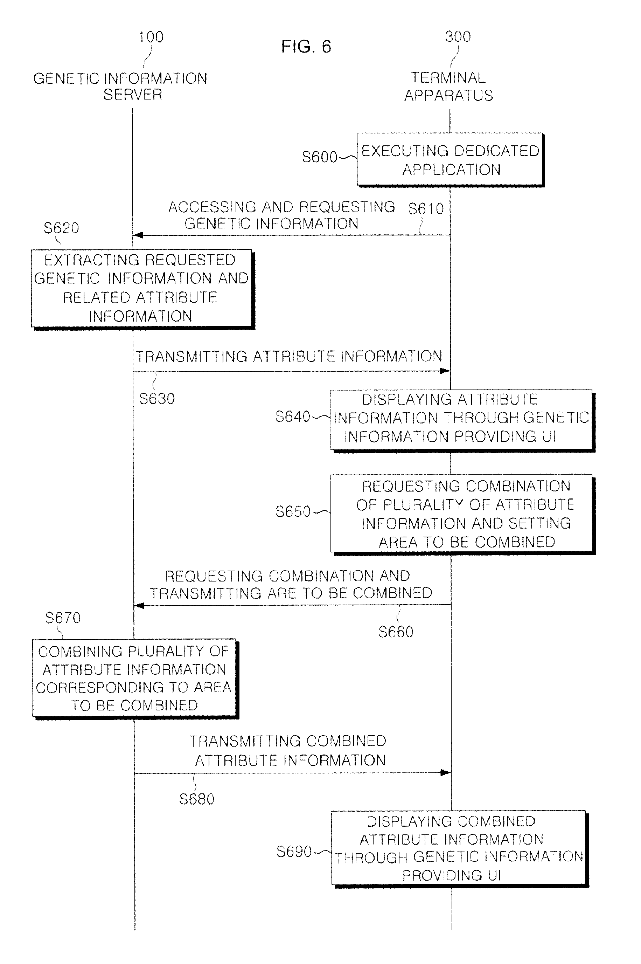

FIG. 6 is a flowchart illustrating a method for providing genetic information according to another exemplary embodiment.

Referring to FIGS. 1 through 4 and FIG. 6, a method for providing genetic information according to another exemplary embodiment will be explained. However, in this embodiment, the terminal apparatus 300 accesses the genetic information server 100 through a dedicated application. The dedicated application may be an application that generally provides access to the genetic information server 100.

The user executes the dedicated application by manipulating the terminal apparatus 300 (S600). Accordingly, the dedicated application displays the genetic information providing Ul 400 in the basic format shown in FIG. 3.

The dedicated application accesses the genetic information server 100 and the user selects genetic information to be provided from the list display area of the genetic information providing Ul 400 of the basic format. After that, the dedicated application requests the genetic information selected by the user from the genetic information server 100 (S610).

Operations S600 and S610 correspond to the case that the genetic information providing Ul 400 already displays the list of the plurality of tracks of genetic information on the list display area 410, if the dedicated application is executed. In this case, the terminal apparatus 300 has already stored the list of the plurality of tracks of genetic information in a memory (not shown) used by the dedicated application.

Alternatively, if the dedicated application is executed, the dedicated application may access the genetic information server 100, obtain the list of genetic information from the genetic information server 100, and display the list on the list display area 410 of the genetic information providing Ul 400. In this case, there is an advantage of reflecting updated genetic information by the genetic information server 100 to the dedicated application promptly.

The genetic information server 100 extracts attribute information regarding the genetic information requested by the terminal apparatus 300 from the genetic information DB 134 (S620), and transmits the extracted attribute information to the terminal apparatus 300 (S630).

The terminal apparatus 300 receives the attribute information from the genetic information server 100. Accordingly, the dedicated application inserts the attribute information received from the genetic information server 100 into the default track of the genetic information providing Ul 400 and displays the genetic information providing Ul 400 on the monitor (S640).

The user can identify the attribute information relating to the genetic information requested by himself/herself through the genetic information providing Ul 400, and, if the user wants to combine some of the attribute information, the user selects attribute information to be combined and requests combination, and sets an area to be combined (S650).

In response to this, the terminal apparatus 300 transmits a user's request for combination to the genetic information server 100 through the dedicated application and the area set to be combined (S660).

The genetic information server 100 combines the plurality of tracks of attribute information corresponding to the area to be combined (S670), and transmits the combined attribute information to the terminal apparatus 300 (S680).

The terminal apparatus 300 receives the combined attribute information from the genetic information server 100, and the dedicated application inserts the combined attribute information into the private track of the genetic information providing Ul 400 and displays the attribute information through the monitor (S690).

Although a subsequent process is not illustrated, additional attribute information relating to different genetic information may be displayed by the processes of FIGS. 5 and 6 even in the case that the attribute information has been already displayed on the default track and the private track. Also, if the default tracks displayed on the screen are insufficient, an additional default track may be generated. Also, an additional private track may be generated so that more combined attribute information can be displayed.

According to the above process, if the user selects the genetic information from the plurality of tracks of genetic information in order to see the attribute information relating to the genetic information and their relationship, the user can identify the attribute information relating to the corresponding genetic information through the default track easily. Also, if the user wants to analyze by comparing the attribute information of the genetic information displayed on the default track, the user sets the area to be combined so that the plurality of tracks of attribute information overlap each other with respect to the area set by the user. Accordingly, the user can easily identify the correlation between the genetic information and the attribute information.

The methods according to the exemplary embodiments may be realized by a program that can be executed by mobile devices such as computers, smart phones, PDAs, and cellular phones and may be recorded on a non-transitory computer readable medium. The program may be a genetic information browser program.

The non-transitory computer readable medium may include a program command, a data file, and a data structure solitarily or in combination. The program command recorded on the medium may be specifically designed and configured to achieve the inventive concept or may be well known to an ordinary skilled person in the computer software field and may be easily used.

While exemplary embodiments have been particularly shown and described above, it will be understood by those of ordinary skill in the art that various changes in form and details may be made therein without departing from the spirit and scope of the inventive concept as defined by the following claims.

## Claims

1. A method for providing genetic information, the method comprising:
displaying a plurality of tracks of attribute information relating to a plurality of tracks of genetic information on a plurality of default tracks, respectively;
selecting at least two tracks from the plurality of tracks of attribute information displayed on the default tracks; and
combining the selected at least two tracks of attribute information and displaying the combined attribute information on a private track.

2. The method as claimed in claim 1, further comprising:
displaying a selectable list of the plurality of tracks of genetic information; and
selecting at least two tracks of genetic information from the list of the plurality of tracks of genetic information,
wherein the displaying the plurality of tracks of attribute information on the plurality of default tracks, respectively, comprises displaying attribute information relating to the selected at least two tracks of genetic information on the default tracks.

3. The method as claimed in claim 1, wherein the displaying the combined attribute information on the private track comprises overlapping the at least two tracks of attribute information.

4. The method as claimed in claim 1, further comprising:
receiving an input of an area to be combined on at least some of the plurality of default tracks,
wherein the displaying the combined attribute information on the private track comprises overlapping the at least two tracks of attributed information corresponding to the input area to be combined.

5. A genetic information apparatus comprising:
a communication unit which transmits a plurality of tracks of attribute information relating to a plurality of tracks of genetic information to be displayed on a plurality of default tracks to a terminal apparatus; and
a combination unit which, if a request for a combination of at least two tracks of attribute information among the transmitted plurality of tracks of attribute information is received from the terminal apparatus, combines the requested at least two tracks of attribute information

6. The genetic information apparatus of claim 5, further comprising:
a storage unit which stores the plurality of tracks of attribute information relating to the plurality of tracks of genetic information,
wherein, if a request for the plurality of tracks of genetic information is received from the terminal apparatus through the communication unit, attribute information relating to the requested genetic information is extracted from the storage unit and transmitted to the terminal apparatus through the communication unit.

7. The genetic information apparatus of claim 5, wherein the combination unit combines the tracks of attribute information requested to be combined by overlapping the tracks of attribute information requested to be combined.

8. The genetic information apparatus of claim 5, wherein the communication unit transmits a dedicated application for providing genetic information to the terminal apparatus.

9. The genetic information apparatus of claim 8, wherein the dedicated application provides a genetic information providing UI that is divided into a list display area to display a selectable list of the plurality of tracks of genetic, a default track area to display the plurality of default tracks, and a private track area to display a private track.

10. The genetic information apparatus of claim 9, wherein the combined attribute information is displayed on the private track by the dedicated application.

11. The genetic information apparatus of claim 5, wherein the communication unit transmits the attribute information through a web browser.

12. The genetic information apparatus of claim 11, wherein the web browser comprises a list display area to display a selectable list of the plurality of genetic information, a default track area to display the plurality of default tracks, and a private track area to display a private track.

13. The genetic information apparatus of claim 12, wherein the combined attribute information is transmitted while being included in the private track area of the web browser.

14. The genetic information apparatus of claim 5, further comprising: a controller which transmits the combined attribute information to the terminal apparatus through the communication unit.

15. A non-transitory computer readable medium having recorded thereon a computer program for executing the method of claim 1.

16. A method for providing genetic information, the method comprising:
generating a private track on which a user can define and customize genetic information; and
displaying genetic information defined and customized by a user on the generated private track.
